# EUROPEAN PATENT APPLICATION

(11) **EP 3 032 267 A2**
(43) Date of publication of application: **15.06.2016**
(21) Application number: 15198623.9
(22) Date of filing: 09.12.2015
(51) Int. Cl.: G01Q 60/42

(54) **METHODS OF ANALYZING SAMPLE SURFACES USING A SCANNING PROBE MICROSCOPE AND SCANNING PROBE MICROSCOPES THEREFOR**

(30) Priority: 10.12.2014 KR 20140177823
(71) Applicant: Samsung Electronics Co., Ltd, Gyeonggi-do 16677 (KR)
(72) Inventor: KWON, Youngnam, 16678 Gyeonggi-do (KR); PARK, Sungjun, 16678 Gyeonggi-do (KR); PAEK, Woonjung, 16678 Gyeonggi-do (KR); KIM, Seongheon, 16678 Gyeonggi-do (KR); AHN, Jaemin, 16678 Gyeonggi-do (KR); YUN, Dongjin, 16678 Gyeonggi-do (KR)
(74) Representative: Greene, Simon Kenneth

(57) **Abstract**

Provided are methods for analyzing a surface of a sample using a scanning probe microscope including a cell-attached probe and scanning probe microscopes therefor.

## Description

### Field of the Invention

The present disclosure relates to a method of analyzing a surface of a sample using a scanning probe microscope and a scanning probe microscope therefor.

### Background of the Invention

Atomic force microscopy (AFM) is a method that is widely used in various industries and fields of research to scan a surface with the aid of an ultra-sharp measurement tip. The measurement tip is located at the unsupported end of a micromechanical cantilever and reacts to short-ranged forces (e.g., van der Waals force). The AFM method is frequently used in the fields of molecular biology, pharmacology, material science and nanotechnology. Furthermore, AFM is industrially utilized on the one hand for process control, but also on the other hand for the study of novel phenomena that play an increasingly important role, in the context of miniaturization and the use of highly integrated circuits.

### Summary of the Invention

An aspect provides a method of analyzing a surface of a sample using a scanning probe microscope.

Another aspect provides a scanning probe microscope.

Another aspect provides a probe for detecting information about a surface of a sample.

An aspect provides a method of analyzing a surface of a sample using a scanning probe microscope, including the steps of providing a prove including a cantilever and a cell attached thereto; moving the probe relative to the sample surface, e.g., using a scanner, to allow interaction between the sample surface and the probe; measuring a deflection distance of the probe using a deflection sensor; and determining, e.g., using a processor of the microscope, an interaction force between the probe and the surface based on the deflection distance.

The method may further include providing a sample having the sample surface, e.g., prior to the step of providing a probe. The method may further include comparing the determined interaction force with a predetermined interaction force.

In the method, the scanning probe microscope includes the probe for detecting information about the surface of the sample, the scanner for moving the probe relative to the sample to allow the probe to scan the surface of the sample, and the deflection sensor for detecting a deflection of the probe, wherein the probe includes with one end connected to the scanner and the other end to which a cell is attached, and the deflection sensor includes a light source that is positioned or located to irradiate light onto the probe and a position-sensitive photodetector that is positioned or located to detect the light reflected from the probe. The scanning probe microscope may be an atomic force microscope (AFM).

The cantilever may be a substance having elasticity. The cantilever may have a spring constant of from about 0.001 N/m to about 1 N/m. The cantilever may be made of silicon or silicon nitride (Si₃N₄). The cantilever may have a thickness of from about 1 nm to about 100 nm. The cantilever may have a length of from about 50 um to about 200 um, e.g., 125 um. The cantilever may have no tip at the end of the probe of a typical AFM, that is, it may have a tipless tip or end. The cantilever may be arranged by a light beam produced by the light source.

The probe deflection sensor may be a cantilever deflection sensor. In the cantilever deflection sensor, the light source may be a laser. The position-sensitive photodetector may be a split photodiode. The laser beam reflected from the back of the cantilever may be directed onto the split photodiode which detects small changes in the deflection of the cantilever.

The scanner may include a mechanism or tool capable of moving the probe or the sample surface or both so that the sample surface and the probe move relative to each other in a controlled manner. The scanner may include a piezo-electric element. The piezo-electric element may be an expansion element. The scanner may include a servo motor for moving the probe and/or the sample surface relative to the other.

Optionally, the method includes the step of coating the sample surface with the anti-biofouling material, e.g., prior to the step of providing the sample having the surface. This step is to prepare the sample surface having anti-biofouling property by reacting the sample surface with the anti-biofouling material. The reaction includes incubating them under conditions that allow contact of the surface with the anti-biofouling material. The reaction includes incubating them after the sample surface is put in a container containing the anti-biofouling material. The anti-biofouling material may show a small adhesion force or a large repelling force with respect to the cell, compared to the sample surface.

The method includes the step of moving the probe relative to the sample surface using the scanner to allow interaction of the sample surface with the probe. The movement of the probe relative to the sample surface by the scanner includes to approach or retract the probe relative to the surface, for example, vertically. In addition, the movement may include to move the probe in the plane, that is, in the x and y directions relative to (e.g., parallel to) the surface. The interaction includes attraction or binding by an adhesion force or retraction by a repelling force.

The movement of the probe relative to the sample surface by the scanner may be performed in air or under surrounding atmosphere, which, in certain aspects is not liquid. The movement may be performed to move the probe and the sample surface relative to each other at a distance ranging from about 0 to about 500 nm depending on the sensitivity of the probe.

The method also includes the step of measuring a deflection distance of the probe using the deflection sensor. The laser beam reflected from the back or surface of the cantilever may be directed onto the split photodiode which detects small changes in the deflection of the cantilever, thereby providing a measure of the deflection distance.

The method includes the step of determining an interaction force between the probe and the surface based on the deflection distance. The interaction force may be an interaction force between the cells attached to the probe and the surface. To convert the deflection distance to the force, it is necessary to define the spring constant of the cantilever and the zero of force. The zero of force may be a point at which the cell-attached probe and the surface are too far apart and thus the deflection is independent of a position, for example, a piezo position. According to an embodiment, the force may be calculated as follows.

The variable to be measured is the voltage, which is corrected by the intrinsic constant value of the tip and the detection constant value of the piezo-electric element responding to the tip, thereby calculating the force. That is, the photodetector measures the V value as a signal, and V is converted to the cantilever deflection distance (nm), which is converted to the force (nN).

F=kx =Vx * deflection sensitivity * k (Equation 1) wherein F is the force, k is the spring constant, x is measured as the deflection distance of the tip, V is the voltage, and deflection sensitivity is the deflection distance (nm) per voltage of the piezo-electric element of SPM scanner with respect to each tip.

The method may include the step of comparing the determined interaction force with a predetermined interaction force. The term "predetermined value" may be an adhesion force or a repelling force between the known anti-biofouling material and the cells attached to the probe. The adhesion force or repelling force may be measured by the above method. In the step of comparing the determined interaction force with the predetermined interaction force, the predetermined interaction force may be an interaction force with respect to the sample surface before the coating step.

The method may further include the step of recognizing the sample surface to have an anti-biofouling property, if the interaction force is an adhesion force and the determined interaction force is smaller than the predetermined interaction force; or the step of recognizing the sample surface to have an anti-biofouling property, if the interaction force is a repelling force and the determined interaction force is larger than the predetermined interaction force. In addition, the method may further include the step of recognizing the sample surface to have a non-anti-biofouling property, if the interaction force is an adhesion force and the determined interaction force is larger than the predetermined interaction force; or the step of recognizing the sample surface to have a non-anti-biofouling property, if the interaction force is a repelling force and the determined interaction force is smaller than the predetermined interaction force. In this case, the method may further include the step of recognizing the sample surface as a defective surface. That is, the analysis is to determine whether the surface treatment of a product is failed or not.

Another aspect provides a scanning probe microscope including a probe for detecting information about the surface of the sample, the probe including a cantilever and a cell attached thereto; a scanner for moving the probe relative to the sample to allow the probe to scan the surface of the sample; and a deflection sensor for detecting deflection of the probe.

In the scanning probe microscope, the deflection sensor may include a light source that is positioned or located to irradiate light onto the probe and a position-sensitive photodetector that is positioned or located to detect the light reflected from the probe, and the scanner may include the expanding piezo-electric element that is connected to the probe.

The scanning probe microscope may be an atomic force microscopy (AFM). The cells may be immobilized by electrostatic binding on the probe, which may be coated with poly(diallyldimethylammonium chloride) (polyDADMAC).

The scanning probe microscope may include a plurality of probes which detects information about different regions of the sample surface and a set of deflection sensors which detects each deflection of a plurality of probes, respectively. A plurality of probes may be arranged relative to the substrate at predetermined intervals.

The elements of the scanning probe microscope, unless otherwise mentioned, have the same meanings as those of the scanning probe microscope which is cited in the above method.

Another aspect provides a probe for detecting information about a surface of a sample, including a cantilever including a cell attached to one end thereof.

In the probe, the cell may be attached to a cationic polyelectrolyte coated on the end of the cantilever. The cationic polyelectrolyte may be poly(diallyldimethylammonium chloride) (polyDADMAC). The cell may be E.coli.

The sample surface may be efficiently analyzed by the method of analyzing the sample surface using the scanning probe microscope according to an aspect.

The scanning probe microscope according to another aspect may be efficiently used in the method of analyzing the sample surface.

### Brief description of the Drawings

These and/or other aspects will become apparent and more readily appreciated from the following description of the exemplary embodiments, taken in conjunction with the accompanying drawings in which:
FIG. 1 is a view illustrating an exemplary embodiment of a scanning probe microscope;
FIG. 2 is an enlarged view of a cantilever deflection sensor;
FIGS. 3A through 3D show results of observing E. coli attached on a poly(diallyldimethylammonium chloride)-coated cantilever with an optical microscope;
FIGS. 4A through 4D show results of examining the size of E. coli by using an scanning electron microscope (SEM) in order to measure the size of microorganism with high resolution at respective magnifications, after observation of E. coli attached on a cantilever with an optical microscope;
FIG. 5 shows measurement results that are obtained by using XPS (X-ray photoelectron spectroscopy) or a plastic surface which is coated with antimicrobial silver nanoparticles;
FIGS. 6A through 6H show experimental procedures carried out in exemplary embodiments;
FIG. 7 shows adhesion forces of various surfaces, which are measured with AFM using different probes;
FIGS. 8A through 8H show distance-force curves corresponding to FIG. 7;
FIG. 9 is a distance-force curve showing a strong interaction force between E. coil on a probe and a surface, in which the curve is obtained by measuring the surface using the probe including E. coli attached to a poly(diallyldimethylammonium chloride)-coated cantilever according to a specific embodiment; and
FIG. 10 is a distance-force curve showing a weak interaction force between E. coil on a probe and a surface, in which the curve is obtained by measuring the surface using the probe including E. coli attached to the poly(diallyldimethylammonium chloride)-coated cantilever according to a specific embodiment.

### Detailed Description

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein.

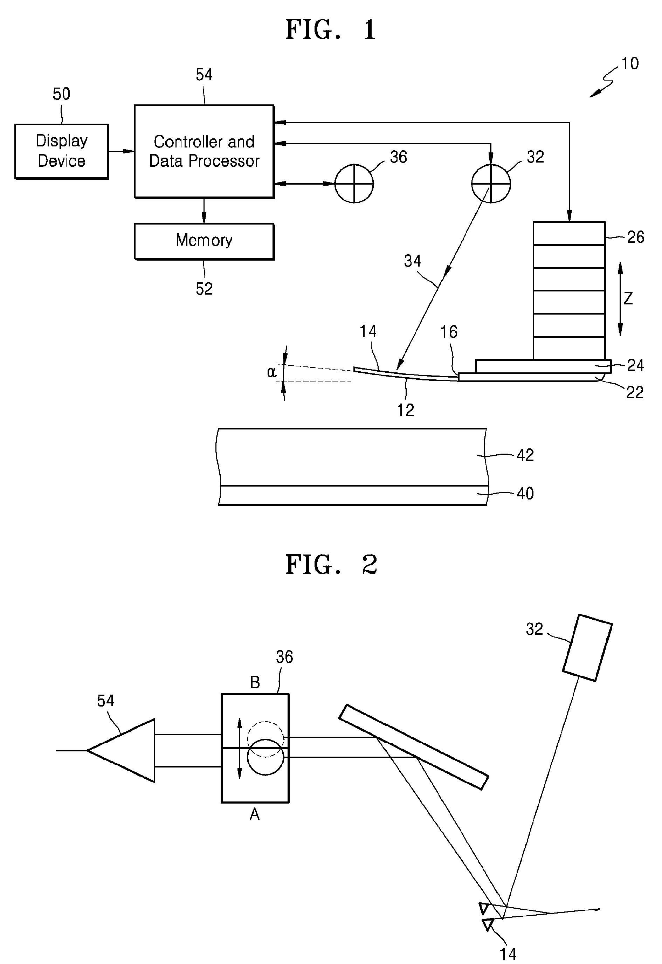

FIG. 1 is a diagram showing an exemplary embodiment of the scanning probe microscope. As shown in FIG. 1, the scanning probe microscope 10 may have a plurality of components, e.g., including certain components typically included in an AFM system. A sample having a surface 42 is placed and supported on a substrate 40. The substrate 40 may be an optically transparent or translucent substance, such as glass or plastic. The scanning probe microscope 10 includes a cantilever 12 having one end 16 that is connected to a cantilever body 22 connected to a cantilever support 24. The cantilever support 24, in turn, is connected to an expansion element 26, such as one or more piezo-electric elements. The expansion element 26 is actuated to expand or contract so as to move the cantilever support 24 or other connected components along the z-axis, for example, vertically. The other end 14 of the cantilever 12 includes cells attached thereto, and thus functions as a probe.

During operation of the scanning probe microscope 10, the cantilever support 24 is moved downwardly as a result of actuating the expansion element 26. Eventually, the end 14 of the cantilever is brought near the sample and interacts with the surface 42 of the sample. When a repelling force or an adhesion force is present or active, the end 14 tilts at a certain angle α relative to a horizontal plane, depending on the amount or intensity of the repelling or adhesion force. The extent of deflection of the cantilever is detected using a light source 32 and a photodetector 36. The light source 32, in one embodiment, includes a laser, which emits a light beam 34 that is brought to focus on an upper surface of the cantilever 12. The light beam 34 is reflected towards and impinges upon or strikes the photodetector 36 as a laser spot. Deflection of the cantilever 12 moves the position of the laser spot on the photodetector 36. The photodetector 36 may be communicably connected to a data processing system including a controller and data processor 54 (which may include one or more microprocessors or processing circuits, e.g., ASICs), a memory 52, and a display device 50. Other mechanisms for detecting the deflection of the cantilever 12 are also contemplated. For example, bending of the cantilever 12 may be detected using an interference-detector element, which detects the extent of interference between a reflected light beam and an original light beam. As another example, a separate piezo-resistive element or a piezo-electric element may be included within or connected to the cantilever 12 so as to detect the extent of bending of the cantilever 12.

As illustrated in FIG. 1, the scanning probe microscope 10 may include a controller and data processor 54, which is connected to various components of the scanning probe microscope 10 and serves to direct or control operation of those components. The controller and data processor 54 is connected to a display device 50, which produces visual indications for a user of the scanning probe microscope 10. The controller and data processor 54 may be also connected to a memory 52 (and/or other non-transitory computer-readable media, such as a hard drive, RAM, ROM, removable/portable media such as an optical disc, etc), which stores computer code or executable instructions for performing various data retrieval and manipulation operations, including those described herein. The memory 52 may also store data including deflection data and data associated with a fail or pass basis of the sample.

FIG. 2 is an enlarged view of the cantilever deflection sensor. As illustrated in FIG. 2, the cantilever deflection sensor includes the light source 32 and the photodetector 36. The light beam emitted from the light source 32 is brought to focus on the end 14 of the cantilever 12, which is deflected by interaction between the end 14 of the cantilever 12 and the surface 42 of the sample. The deflection is detected by the photodetector 36, e.g., as a position of laser spots A and B and/or movement of the laser spot A to B, or vice versa, on the photodetector. The difference or sum of the laser spots represents the deflection extent of the cantilever.

In an embodiment, cells may be attached to the cantilever end 14, e.g., immobilized by electrostatic binding on the surface of the probe, which may be coated with a cationic polyelectrolyte, for example, poly(diallyldimethylammonium chloride) (polyDADMAC). The cells may be bacterial or mammalian cells. The bacteria may be Gram-positive or Gram-negative. The bacteria may be, for example, E. coli, salmonella, or pseudomonas. The cells may be immobilized on the probe as viable cells or as physiologically active cells.

In an embodiment, a method of analyzing a surface of a sample is provided. At step A the sample having the sample surface is provided. At step B, a probe is moved relative to the sample surface using a scanner to allow interaction between the sample surface and the probe. The scanner is configured to move the probe relative to the sample to allow the probe to scan the sample surface. At step C, a deflection distance of the probe is measured using a deflection sensor. At step D, using a processor of the scanning probe microscope, an interaction force between the probe and the sample surface is determined based on the deflection distance. In step E, using the processor, the determined interaction force is compared with a predetermined interaction force. The probe includes a cantilever with one end connected to the scanner and the other end to which a cell is attached. The deflection sensor includes a light source that is positioned to irradiate light onto the other end of the cantilever and a position-sensitive photodetector that is positioned to detect the light reflected from the other end of the cantilever. Steps B through E may be repeated one or more time to evaluate different regions of the surface of the sample or different samples.

In an embodiment, the scanning probe microscope includes a plurality of probes each of which detects information about a different surface region of the sample, or different sample surfaces, and a set of deflection sensors which detects each deflection of the plurality of probes, and the above steps may be performed at the same time with respect to a plurality of locations or regions on the sample surface or sample surfaces. For example, a plurality of probes may be fixed relative to the surface of the same substrate of the scanner, and thus allows for movement from a different position of the sample surface at the same time. In this case, analysis of the sample surface may be more time-efficient. The deflection sensor may include one or more light sources which may be disposed or adjusted to be disposed so as to radiate light onto the sample surface. The number of light sources may be, for example, 2 or more, 3 or more, or 4 or more. Light emitted by one or more of the light sources may be split one or more times (e.g., using dichroic beam splitter elements or other beam splitting elements) so that fewer sources may be used to generate multiple light beams.

In an embodiment, a plurality of probes in the scanning probe microscope may be arranged relative to the substrate at predetermined intervals. The sample surface may be uniformly analyzed by arranging the individual probes at predetermined intervals.

In an embodiment, the method includes the step of providing a sample having a surface. The sample includes any substance, as long as it has a surface. The sample may be a solid substance having a flat surface. The sample may be a cell phone (e.g., display screen surface), a household appliance or item, or a vehicle having a plastic display surface. The sample may have a surface onto which an anti-biofouling material is attached, coated, or applied, indirectly or directly, or embedded or integrated. An adhesion force between the "anti-biofouling material" and the cells attached to the probe may be smaller than a predetermined value or a repelling force therebetween may be larger than the predetermined value. The "predetermined value" may be an adhesion force or repelling force between a known anti-biofouling material and the cells attached to the probe. The cells may be from bacteria, yeast, barnacles, bryozoans, mollusks, polychaetes, and/or zebra mussels. The cells may be from seaweeds, hydroids, algae, or a combination thereof. The cells may be Gram-negative bacteria. The anti-biofouling material may be, for example, an antibiotic or antibiotic-incorporated material (e.g., antibiotic-incorporated plastic material), a biocide or biocide-incorporated material (e.g., biocide-incorporated copper acrylate self-polishing copolymer), a silver nanomaterial or silver nanomaterial-coated material, a self-assembled block copolymer, or a fluorinated block copolymer. In addition, the anti-biofouling material may have low friction and low surface energy. This material may allow the surface to be hydrophobic. The hydrophobic surface may create a smooth surface capable of preventing microbial adhesion. The anti-biofouling material may be a fluoropolymer and/or a silicone. The silicone may include polydimethylsiloxane (PDMS). The fluoropolymer may include polytetrafluoroethylene (PTFE), polyvinyl fluoride (PVF), polyvinylidene fluoride (PVDF), polyhexafluoropropylene, fluorinated ethylene propylene, perfluoropolyether (PFPE), polychlorotrifluoroethylene (PCTFE), polyethylene chlorotrifluoroethylene (ECTFE), polyethylene tetrafluoroethylene (ETFE), or the like, or a polymer comprising two or more of any of these. The PFPE may be, for example, KRYTOX lubricant family manufactured by DuPont (e.g., DupontTM Krytox® 100 or Dupont™ Krytox® 103), or perfluoropolyether(PFPE) silane. The PFPE may have a water contact angle of about 115° and a friction coefficient of about 0.1 to about 0.15. "Perfluorinated" group represents a group or a compound in which all C-H bonds are replaced by C-F bonds. "Ether" represents a group or a compound having an oxy group between two carbon atoms. Ether group is often a divalent group such as -CH₂-O-CH₂- or -CF₂-O-CF₂-. The term "polyether" represents a group or a compound having a plurality of ether groups. Perfluoropolyether (PFPE) silane represents perfluoropolyether (PFPE) having a silyl group which reacts with the surface of the siliceous substrate to form a - Si-O-Si- bond. The perfluoropolyether (PFPE) silane may be, for example, those having the following Chemical Formulae I and II.

CF₃O(CF₂CF₂O)ₘ(CF₂O)ₙCF₂-CH₂O-(CH₂)ₚ-Si(R¹)₃₋ₓ(R²)ₓ (Formula I)

F(CF(CF₃)CF₂O)ₘCF(CF₃)-CH₂O-(CH₂)ₚ-Si(R¹)₃₋ₓ(R²)ₓ (Formula II)

In Formulae I and II, R¹ is a hydroxy or hydrolyzable group, R² is a non-hydrolyzable group, and variable X is 0, 1, or 2. Variable n or m is an integer of 4 to 100, 5 to 100, 10 to 100, 10 to 80, 10 to 60, 10 to 50, 10 to 40, 20 to 100, 40 to 100, 50 to 100, or 60 to 100. Variable p is an integer of 1 to 6, 1 to 4, or 1 to 3. The "hydrolyzable group" represents a group capable of reacting with water at pH 1 to 10 under atmospheric pressure. When the hydrolyzable group reacts, it is usually converted to a hydroxyl group. The hydroxyl group may undergo an additional reaction, such as reaction with the siliceous substrate. The hydrolyzable group may include an alkoxy, aryloxy, aralkyloxy, acyloxy, and/or halo group.

Alkoxy R¹ may have a formula of -OR^{a} in which R^{a} is an alkyl group having 1 to 10 carbon atoms, 1 to 6 carbon atoms, 1 to 4 carbon atoms, 1 to 3 carbon atoms, or 1 to 2 carbon atoms. The alkyl moiety of the alkoxy group may be linear, branched, or cyclic, or a combination thereof.

Aryloxy R¹ may have a formula of -OAr in which Ar is an aryl group. The aryl group is a monovalent group having one or more carbocyclic aromatic rings, optionally including one or more heteroatoms, such as N, S, and/or O. Additional carbocyclic rings may be fused to the aromatic rings. Any additional ring may be unsaturated, partially saturated, or saturated. The aryl moiety of the aryloxy group may have 6 to 12 carbon atoms, or 6 to 10 carbon atoms. In many specific embodiments, the aryloxy group may be phenoxy.

Aralkyloxy R¹ may have a formula of -OR^{b}-Ar in which R^{b} may be a divalent alkylene group (namely, divalent radical of alkyl) having 1 to 10 carbon atoms, 1 to 6 carbon atoms, or 1 to 4 carbon atoms, and Ar group is an aryl group having one or more carbocyclic aromatic rings, optionally including one or more heteroatoms, such as N, S, and/or O. Additional carbocyclic rings may be fused to the aromatic rings. Any additional ring may be unsaturated, partially saturated, or saturated. The aryl moiety of the aryloxy group may have 6 to 12 carbon atoms, or 6 to 10 carbon atoms. In specific embodiments, the aryl group may be phenyl.

Acyloxy R¹ may have a formula of -O(CO)R^{c} in which R^{c} is alkyl, aryl, or aralkyl, (CO) group represents a carbonyl group, and alkyl R^{c} has 1 to 10 carbon atoms, or 1 to 6 carbon atoms. Aryl R^{c} is carbocyclic and has one or more aromatic rings, optionally including one or more heteroatoms, such as N, S, and/or O. Additional carbocyclic rings may be fused to the aromatic rings. Any additional ring may be unsaturated, partially saturated, or saturated. The aryl group may have 6 to 12 carbon atoms, or 6 to 10 carbon atoms. In many specific embodiments, the aryl group may be phenyl. Aralkyl R^{c} may have an alkylene group (namely, divalent radical of alkyl) having 1 to 10 carbon atoms, 1 to 6 carbon atoms, or 1 to 4 carbon atoms, and an aryl group having 6 to 12 carbon atoms or 6 to 10 carbon atoms. The alkylene moiety of the aralkyl group may be linear, branched, or cyclic, or a combination thereof. The aryl moiety of the aralkyl group may have one or more carbocyclic aromatic rings. Additional carbocyclic rings may be fused to the aromatic rings. Any additional ring may be unsaturated, partially saturated, or saturated. The aryl group of the acyloxy group may be phenyl.

Halo group R¹ may be a bromo, iodo, or chloro group.

In Formula I and II, each R² group is a non-hydrolyzable group. The term "non-hydrolyzable group" represents a group that does not react with water at pH 1 to 10 under atmospheric pressure. In a specific embodiment, the non-hydrolyzable group is an alkyl group, an aryl group, or an aralkyl group. Alkyl R² may have 1 to 10 carbon atoms, 1 to 6 carbon atoms, or 1 to 4 carbon atoms. The alkyl group may be linear, branched, or cyclic, or a combination thereof. Aryl R² is carbocyclic and has one or more aromatic rings, optionally including one or more heteroatoms, such as N, S, and/or O. Additional carbocyclic rings may be fused to the aromatic rings. Any additional ring may be unsaturated, partially saturated, or saturated. The aryl group may have 6 to 12 carbon atoms, or 6 to 10 carbon atoms. In many specific embodiments, the aryl group may be phenyl. Aralkyl R² may have an alkylene group (namely, divalent radical of alkyl) having 1 to 10 carbon atoms, 1 to 6 carbon atoms, or 1 to 4 carbon atoms, and an aryl group having 6 to 12 carbon atoms or 6 to 10 carbon atoms. The alkylene moiety of the aralkyl group may be linear, branched, or cyclic, or a combination thereof. The aryl moiety of the aralkyl group may have one or more carbocyclic aromatic rings. Additional carbocyclic rings may be fused to the aromatic rings. Any additional ring may be unsaturated, partially saturated, or saturated. The aryl group of the acyloxy group may be phenyl.

The anti-biofouling material may include a material of the following Formula III.

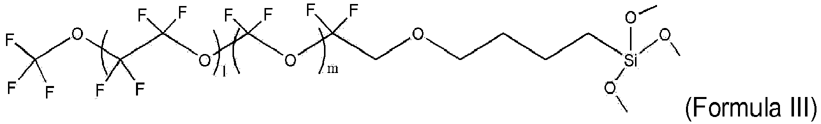

The anti-biofouling material may have a water contact angle of 100 to 150°, 100 to 140°, 100 to 130°, 110 to 150°, or 120 to 150°.

### Example:

### (1) E. coli culture

Gram-negative E. coli used in this Example is DH5α™ competent cell (Life Technologies Korea LLC). One vial of DHSa™ competent cells prepared previously is thawed and plated on a plate, and several colonies are picked up and suspended in 3 mL of Luria Bertani (LB) medium, followed by culture for 12~16 hours. Here, cell density is 1x10⁹ cells (OD>1.8) per ml. This culture broth is subcultured in 200 ml of Luria Bertani fresh medium until OD₆₀₀ reaches 1.6~1.8. Every time, E. coli that is grown until OD₆₀₀ reaches 0.6-1 after about 2-3 subcultures is used. Culture is conducted at 37°C under aerobic conditions. E. coli in the middle of log phase is used.

### (2) Coating of E. coli at end of cantilever in AFM

AFM used in this Example is Dimension Icon (Bruker), and for the surface observation by two-dimensional scanning of the probe, the peak force tapping mode is used to repeat a series of operations including application of a force to a sample and separation therefrom by moving a probe in the vertical direction. Because a force curve is obtained every 1 cycle, its interpretation provides mapping of elasticity or adhesion force and kinetic properties as well as the surface shape.

The cantilever of AFM is made of Si₃N₄ and its spring constant is 0.05 N/m. This spring constant is used for calculating the force. The cantilever is a tipless cantilever having no tip at the other end. The cantilever has a thickness of 4 µm and a length of 125 um.

First, the end of the cantilever is coated with poly(diallyldimethylammonium chloride) (Sigma Aldrich, cat. no.26062-79-3) having Formula 2.

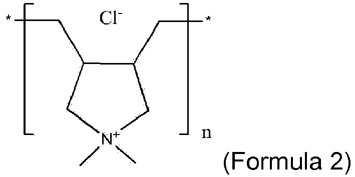

In detail, 35wt.% poly(diallyldimethylammonium chloride) solution (average Mw <100,000) in water is diluted 2,000-fold with water, and 10 µl of the diluted solution is loaded on the cantilever, which is incubated at room temperature for 30 minutes to 1 hour to provide a sufficient time for the diluted solution at the end of the tip to function as an adhesive between the microbe and the SPM tip.

Next, the cantilever is washed with 10 ml of distilled water three times and then dried.

E. coli DH5α™ competent cells cultured in LB medium are precipitated using a centrifuge, the supernatant is discarded and only cells are collected and washed with distilled water two or three times, and then prepared at a density of 10⁶ cell/ml and 10⁵ cell/ml. 10 µl thereof is loaded on the end of the poly(diallyldimethylammonium chloride)-coated cantilever thus obtained, which is incubated at room temperature for 30 minutes to 1 hour to allow the cells to bind to the adhesive poly(diallyldimethylammonium chloride). The cantilever is washed with 10 ml of distilled water three times. As a result, the E. coli-coated cantilever is obtained.

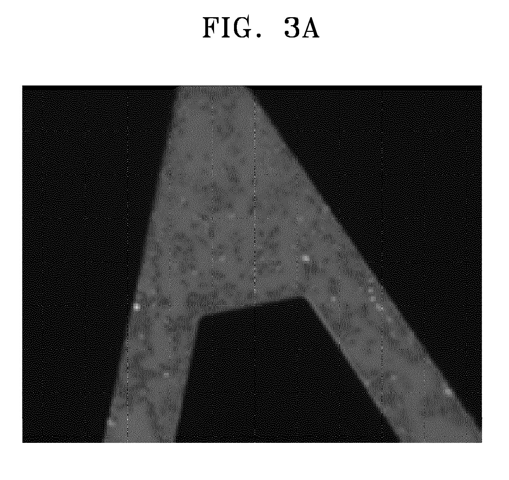

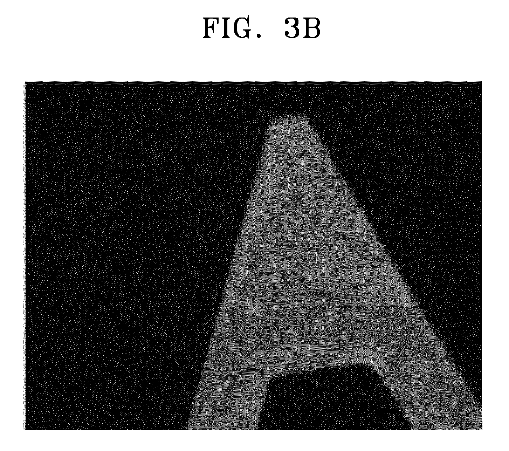

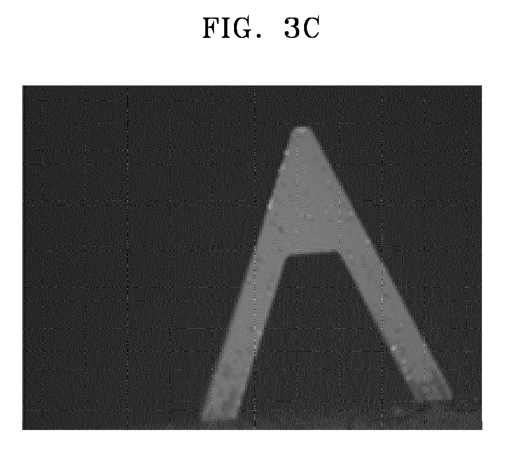

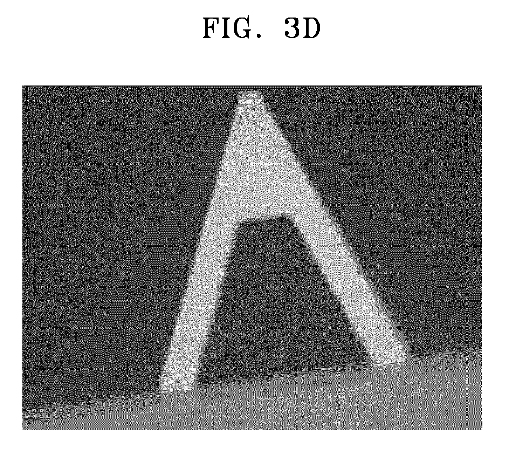

FIG. 3 shows the results of observing E. coli which is attached on the poly(diallyldimethylammonium chloride)-coated cantilever by an optical microscope. It could be observed using the optical microscope without staining. In FIG. 3, (A) and (B) represent the E. coli-coated cantilever obtained by contacting the cantilever with E.coli having density of 10⁶ cell/ml, magnification 100X, (C) represents the E. coli-coated cantilever obtained by contacting the cantilever with E.coli having density of 10⁵ cell/ml, magnification 10X, and (D) represents control cantilever obtained by contacting the cantilever with a medium without containing E.coli, magnification 10X.

FIG. 3 shows the results of primary observation of the microorganism by adjusting two-different concentration level using the optical microscope without staining, in order to examine whether E. coli at each concentration is well-attached on the poly(diallyldimethylammonium chloride)-coated cantilever.

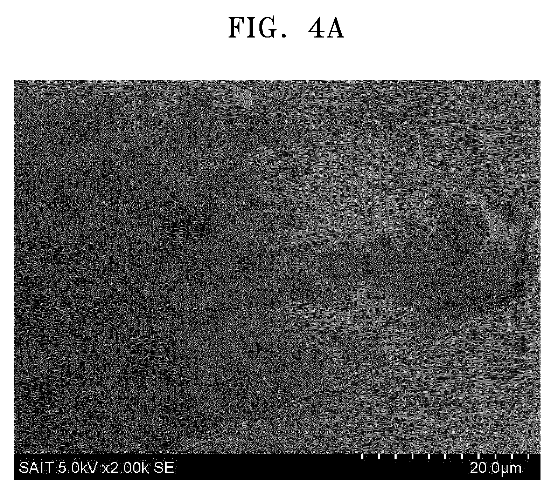

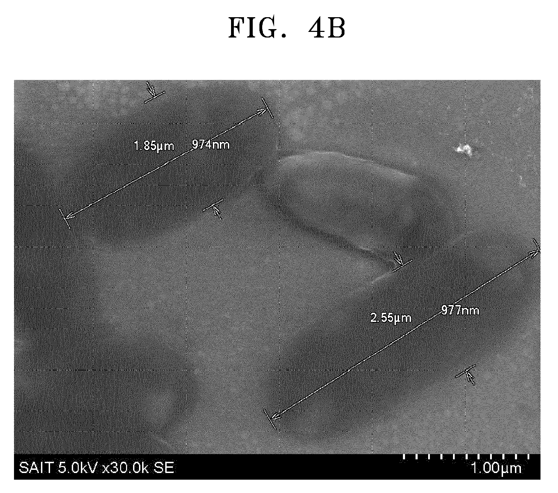

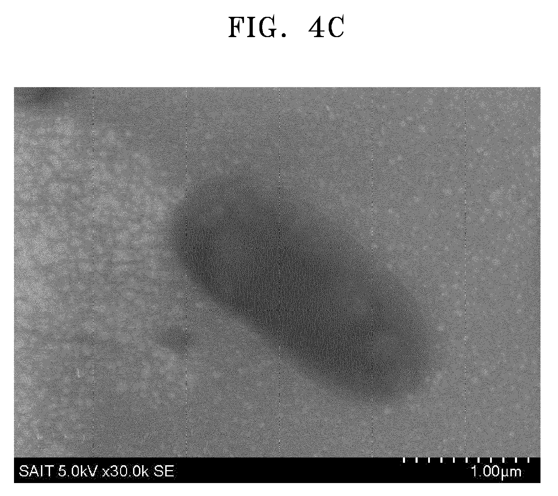

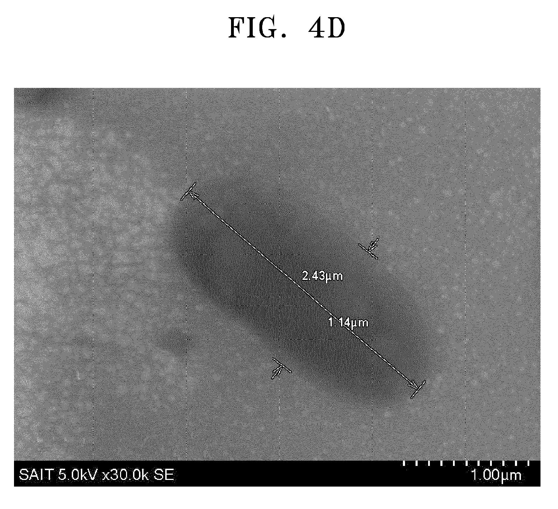

FIG. 4 shows the results of examining the size of E. coli by a scanning electron microscope (SEM) in order to measure the size of the microorganism with high resolution at each magnification, after observation of E. coli attached on the cantilever by the optical microscope. In FIG. 4, A, B, C, and D are the results at 1,500X magnification (A, B, and C), and 30,000X magnification (D) for the area of 2.5 um X 1.04 um, respectively. According to FIG. 4, E. coli is attached on the cantilever with maintaining its intact morphology.

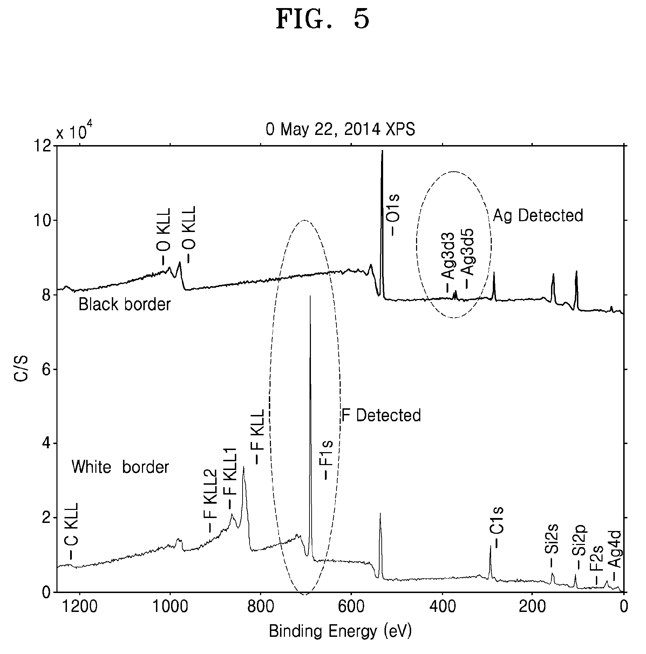

FIG. 5 shows the results of XPS for a plastic surface which is coated with antimicrobial silver nanoparticles. XPS analysis instrument is Quantum 2000 (ULVAC-PHI Inc.) (beam source: Alkα(hv= 1486.6 eV) and Beam size: 100 um).

As shown in FIG. 5, F and Ag components are detected, suggesting that the surface has an anti-biofouling property. XPS instrument can be used to analyze chemical components of the surface of 10 nm or less. Since each element has a unique set of binding energies, information about the component and chemical composition can be identified by analysis of binding energy and intensity of each peak.

In FIG. 5, the y axis represents electron counts in arbitrary units, and x axis represents binding energy (eV). In addition, the line labeled as black border represents a case where only Ag nanomaterial layer is coated on the glass surface and the line labeled as white border represents a case where only anti-fingerprint organic compound layer is coated on the glass surface.

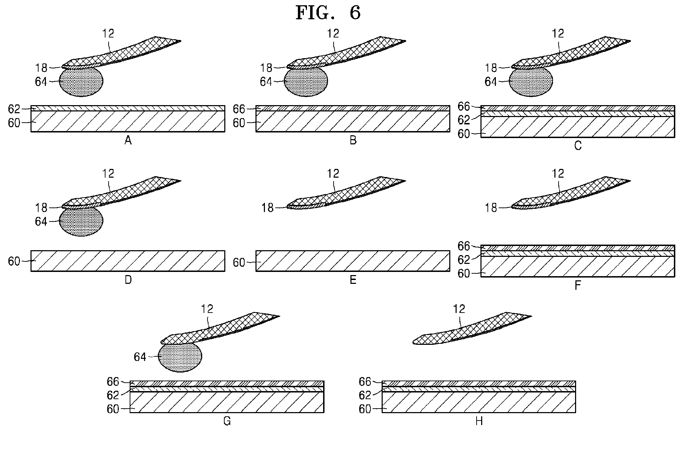

FIG. 6 shows the experimental procedures carried out in the Examples. The procedures can be conducted under the ambient condition, i.e., non-liquid condition, such as atmosphere or air, including dry or wet air. In this example, the experiments are conducted in air. In FIG. 6, the surface of the substrate 60 is coated with a silver nanomaterial 62 or an anti-fouling material 66, or it is coated with the silver nanomaterial 62 and then coated with the anti-fouling material 66 thereon, or it is not coated.

FIG. 6A shows measurement of the adhesion force or the repelling force between E. coli attached at the end of the polyDADMAC 18-coated probe (or cantilever 12) and the silver nanomaterial 62-coated substrate which is obtained by coating a silver nanoparticle-impregnated silica layer at a thickness of 10 nm on the surface of the glass substrate 60 by deposition. FIG. 6B shows measurement of the adhesion force or the repelling force between E. coli 64 attached at the end of the polyDADMAC 18-coated probe 12 and the surface 66 which is obtained by coating a silane-introduced anti-fingerprint organic compound, perfluoropolyether silane (PFPE) of Formula III (OPTOOL™-UD502, Daikin) (water contact angle of 115°, friction coefficient of 0.1 to 0.15) at a thickness of several nm on the surface of the glass 60 by deposition. FIG. 6C shows measurement of the adhesion force or the repelling force between E. coli 64 attached at the end of the polyDADMAC 18-coated probe 12 and the surface which is obtained by coating a silane-introduced anti-fingerprint organic compound, silane (OPTOOL™-UD502, Daikin) (water contact angle of 115°, friction coefficient of 0.1 to 0.15) 66 at a thickness of several nm on the silver nanomaterial layer 62 of the surface of the glass substrate 60 of FIG. 6A by deposition. In this case, the total thickness of the silver nanomaterial layer 62 and the anti-fingerprint organic compound layer 66 is less than 20 nm. FIG. 6D shows measurement of the adhesion force or the repelling force between E. coli 64 attached at the end of the polyDADMAC 18-coated probe 12 and the plain glass surface 60 having no silver nanomaterial layer 62 and no anti-fingerprint organic compound layer 66. FIG. 6E shows measurement of the adhesion force or the repelling force between no E. coli-attached polyDADMAC 18-coated probe 12 and the plain glass surface 60 having no silver nanomaterial layer 62 and no anti-fingerprint organic compound layer 66. FIG. 6F shows measurement of the adhesion force or the repelling force between no E. coli-attached polyDADMAC 18-coated probe 12 and the anti-fingerprint organic compound layer 66 coated on the silver nanomaterial layer 62 of FIG. 6C. FIG. 6G shows measurement of the adhesion force or the repelling force between E. coli 64 attached at the end of no polyDADMAC 18-coated probe 12 and the anti-fingerprint organic compound layer 66 coated on the silver nanomaterial layer 62 of FIG. 6C. FIG. 6H shows measurement of the adhesion force or the repelling force between no E. coli 64-attached, no polyDADMAC 18-coated probe 12 and the anti-fingerprint organic compound layer 66 coated on the silver nanomaterial layer 62 of FIG. 6C.

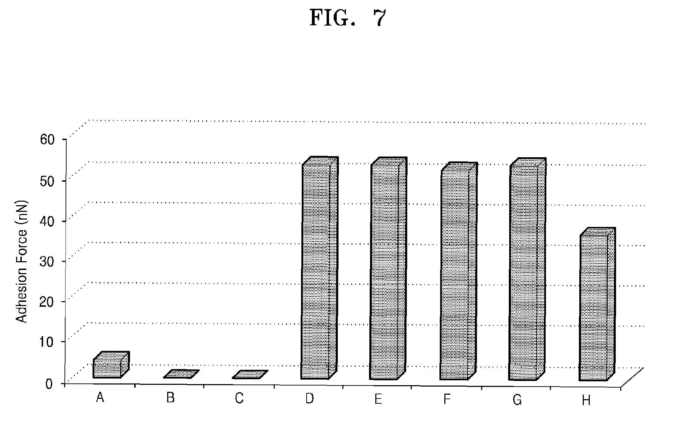

FIG. 7 shows the adhesion forces of various surfaces, which are measured by AFM using different probes. In FIG. 7, A, B, C, D, E, F, G and H represent the adhesion forces measured according to the procedures of A, B, C, D, E, F, G and H of FIG. 6, respectively. Control experiments are performed to examine the effect of the microbe attached at the end of the probe, the effect of the linker between the microbe and the probe, and the effects of the linker and the microbe, respectively.

A, B, and C show small adhesion forces (e.g., adhesion forces of B and C are almost 0 nN), suggesting that the substrates of A, B, and C have the anti-biofouling property. When the microbial probe is brought close (approach) to the surface, it is considered that the repelling force acts on the surface to prevent adhesion of the microbe to the surface. D to G show the adhesion force of 52.2 nN or more, which is out of detection, and H shows the adhesion force of 30 nM or more. In detail, it is considered that D shows no anti-biofouling property because the glass surface coated with no anti-biofouling material shows a strong adhesion force with E. coli. According to E and F, the poly(diallyldimethylammonium chloride) (polyDADMAC) layer shows a strong adhesion force with respect to the glass surface coated with no anti-biofouling material and the anti-fingerprint organic compound layer 66 coated on the silver nanomaterial layer 62. Thus, they are not suitable for determination of anti-biofouling property of the surface. According to H, the cantilever 12 made of silicon nitride shows a strong adhesion force with respect to the anti-fingerprint organic compound layer 66 coated on the silver nanomaterial layer 62, and thus it is not suitable for determination of anti-biofouling property of the surface. Lastly, according to G, when E. coli is attached at the end of the probe without coating with the cationic polyelectrolyte, poly(diallyldimethylammonium chloride) (polyDADMAC), it shows a strong adhesion force with respect to the anti-fingerprint organic compound layer 66 coated on the silver nanomaterial layer 62. Referring to C, in which E. coli attached to the probe coated with the cationic polyelectrolyte, poly(diallyldimethylammonium chloride) (polyDADMAC) shows the adhesion force of about 0 nM with respect to the anti-fingerprint organic compound layer 66 coated on the silver nanomaterial layer 62, it is suggested that E. coli of G is hardly adhered to the surface of the probe 12. Therefore, silicon nitride as the probe material is considered to interact with the anti-fingerprint organic compound layer 66 coated on the silver nanomaterial layer 62 so as to show the strong adhesion force. According to E, F, and H, no E. coli-attached probe 12 is not suitable for determination of anti-biofouling property of the anti-fingerprint organic compound layer 66 coated on the silver nanomaterial layer 62.

The adhesion force shown in FIG. 7 is an average of only the highest values which are obtained by repeated approach and retraction of the probe to 20 points of each substrate.

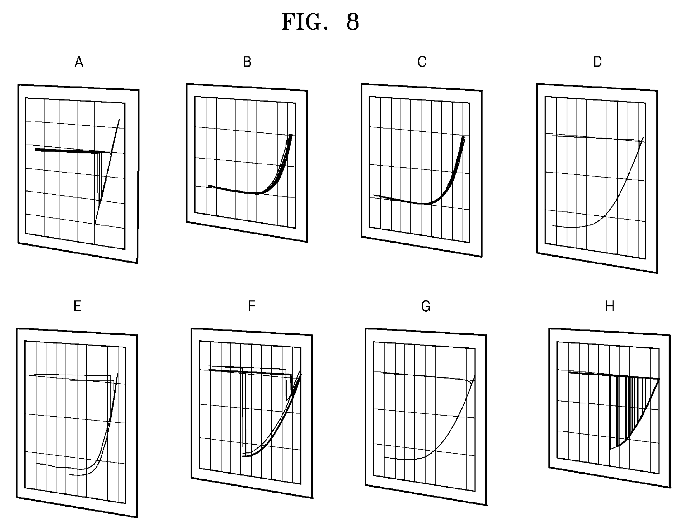

FIG. 8 shows distance-force curves corresponding to FIG. 7. In FIG. 8, the horizontal axis represents distance (nm) and the vertical axis represents adhesion force (V). A,B,C,D,E,F,G, and H of FIG. 8 are the results corresponding to the experiments of A,B,C,D,E,F,G, and H of FIG. 6, respectively.

In FIG. 8, the adhesion force variable voltage may be converted to the unit, nm by the following Equation :

F=kx =Vx times deflection sensitivity times k (Equation 1) wherein F is the force, k is the spring constant (0.05 nm/nm), x is measured as the deflection distance (nm) of the tip, V is the voltage, and deflection sensitivity (87 nm/V) is the deflection distance (nm) per voltage of the piezo-electric element of SPM scanner with respect to each tip.

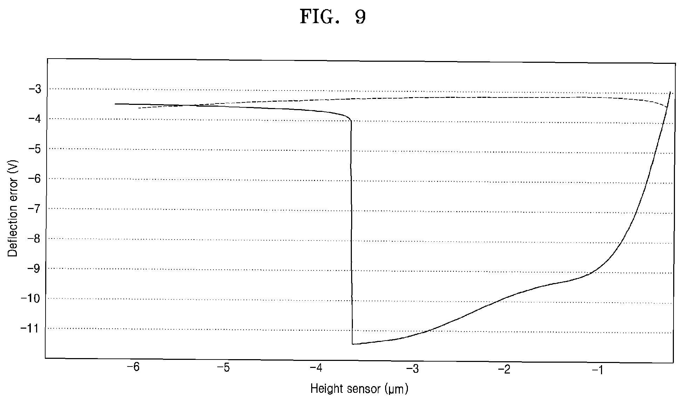

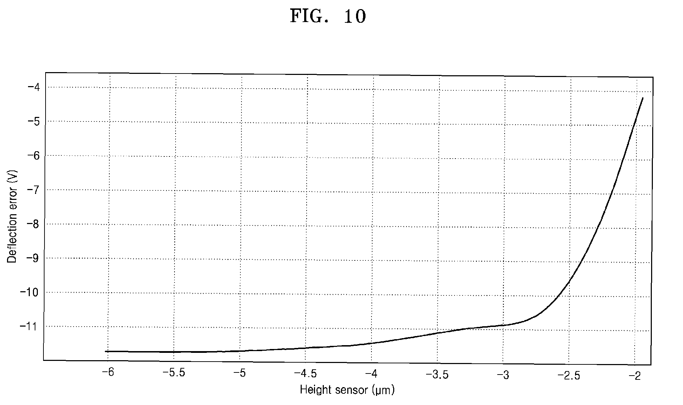

In FIG. 8, the horizontal axis represents the height, namely, the distance (um) returned from the free space after the contact of the probe with the surface. It represents the traveling distance of the probe to evaluate its adhesion force with respect to the surface when the probe returns after movement from the left start point, -7 um to the surface contact point, 0 um. The vertical axis represents the voltage, namely, deflection of the probe when it is retracted after contact with the surface, and also represents the voltage when micromovement of the probe is detected by the photodetector. FIG. 8 shows the values which are obtained by evaluating the surface adhesion force with respect to 20 or more points of the surface in each test, and these values are overlapped for given on a single graph. Strength of the adhesion force may be evaluated even by the shape of the graph. The evaluation method for strength of the adhesion described here will be more clearly described in reference to FIGS. 9 and 10.

FIG. 9 is a distance-force curve showing a strong interaction force between E. coil on the probe and the surface (e.g., D of FIG. 8), in which the curve is obtained by measuring the surface of a specific embodiment using the probe including E. coli attached to the poly(diallyldimethylammonium chloride)-coated cantilever. A curve is similar to FIG. 9 is observed for D to H of FIG. 8.

FIG. 10 is a distance-force curve showing a weak interaction force between E. coil on the probe and the surface (e.g., A of FIG. 8), in which the curve is obtained by measuring the surface of a specific embodiment using the probe including E. coli attached to the poly(diallyldimethylammonium chloride)-coated cantilever. A curve is similar to FIG. 10 is observed for A to C of FIG. 8.

In FIG. 9, the height sensor (um) at the horizontal axis represents the distance that the probe of the scanning probe microscope vertically moves toward the surface of the substrate or the opposite distance. The farthest distance is -7 um. The closest distance is 0 um, that is, the probe and the surface of the substrate being in contact with each other. The deflection error (V) at the vertical axis represents the extent of deflection, which is measured during vertical movement of the probe of the scanning probe microscope.

A distance-force relationship is described with reference to FIGS. 9 and 10, as follows. In FIG. 9, the dotted line represents changes of the voltage (or force) according to approach of the probe tip from -7 um to 0 um (namely, from left to right along the horizontal axis). The solid line represents changes of the voltage (or force) according to separation of the probe tip from the contact with the surface of the substrate (distance 0 um) to -7 um (namely, from right to left along the horizontal axis). In FIG. 9, as the probe tip is separated from the contact with the surface of the substrate, a high voltage is detected, compared to that upon approaching. As in FIG. 9, when they are separated from each other, the voltage is about -11 V to -2 V. Therefore, FIG. 9 indicates a strong adhesion force between the probe and the surface of the substrate. In this case, a difference in the voltages between approach of the probe to the substrate and separation therefrom(e.g., the area between the voltage curve when the probe is approached to the substrate and the voltage curve when the probe is separated therefrom) represents the strength of the adhesion force between the probe and the substrate. In FIG. 10, there is no difference between the voltage curve when the probe is approached to the substrate and the voltage curve when the probe is separated therefrom. Therefore, FIG. 10 indicates a weak adhesion force between the probe and the surface of the substrate.

When the results of FIGS. 8A through 8H are analyzed with reference to FIGS. 9 and 10, FIGS. 8A through 8C show a small area between the voltage curve when the probe is approached to the substrate and the voltage curve when the probe is separated therefrom, suggesting that the adhesion force between the probe and the substrate is not strong. In contrast, FIGS. 8F and 8H show a large area between the voltage curve when the probe is approached to the substrate and the voltage curve when the probe is separated therefrom, suggesting that the adhesion force between the probe and the substrate is strong. In addition, FIGS. 8D, 8E and 8G show that the adhesion force between the probe and the substrate is too strong to separate the probe from the substrate after approaching the probe to the substrate, indicating a very strong adhesion force between the probe and the substrate.

The use of the terms "a" and "an" and "the" and "at least one" and similar referents in the context of describing the embodiments (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely for better illustration and does not pose a limitation on the scope of the disclosure unless otherwise claimed. No language in the specification should be construed as indicating any non-claimed element as essential to the practice the embodiments of the disclosure.

Various embodiments are described herein. Variations of those embodiments may become apparent to those of ordinary skill in the art upon reading the foregoing description. The inventors expect skilled artisans to employ such variations as appropriate, and the inventors intend for the embodiments to be practiced otherwise than as specifically described herein. Accordingly, this specification should be read to include all modifications and equivalents of the subject matter recited in the claims appended hereto. Moreover, any combination of the above-described elements in all possible variations thereof is encompassed by the disclosure unless otherwise indicated herein or otherwise clearly contradicted by context.

## Claims

1. A method of analyzing a surface of a sample using a scanning probe microscope, the method comprising:
providing a probe including a cantilever and a cell attached thereto;
moving the probe relative to a sample surface using a scanner to allow interaction between the sample surface and the probe;
measuring a deflection distance of the probe using a deflection sensor; and
determining an interaction force between the probe and the sample surface based on the deflection distance.

2. The method of claim 1, wherein the scanning probe microscope includes the probe for detecting information about the sample surface, the scanner for moving the probe relative to the sample to allow the probe to scan the sample surface, and the deflection sensor for detecting a deflection of the probe, wherein the probe includes a cantilever with one end connected to the scanner and the other end to which a cell is attached, and the deflection sensor includes a light source that is positioned to irradiate light onto the other end of the cantilever and a position-sensitive photodetector that is positioned to detect the light reflected from the other end of the cantilever

3. The method of claim 1 or 2, further comprising providing a sample having the sample surface.

4. The method of claim 1, 2 or 3 further comprising comparing the determined interaction force with a predetermined interaction force,
and preferably recognizing the sample surface to have anti-biofouling property, if it is determined that the interaction force is an adhesion force and the determined interaction force is smaller than the predetermined interaction force; or recognizing the sample surface to have anti-biofouling property, if it is determined that the interaction force is a repelling force and the determined interaction force is larger than the predetermined interaction force.

5. The method of any preceding claim, wherein the movement of the probe relative to the sample surface by the scanner includes to approach or retract the probe relative to the sample surface,
and preferably wherein the movement of the probe relative to the sample surface by the scanner is performed in air or a atmosphere.

6. The method of any preceding claim, wherein the scanner includes an expanding piezo-electric element that is connected to the probe.

7. The method of any preceding claim, wherein the cell is immobilized by electrostatic binding on the other end of the cantilever coated with a cationic polyelectrolyte, and preferably wherein the cationic polyelectrolyte is poly(diallyldimethylammonium chloride) (polyDADMAC).

8. The method of any preceding claim, wherein the cell includes a viable cell.

9. The method of any preceding claim, wherein the scanning probe microscope is an atomic force microscope (AFM).

10. The method of any preceding claim, wherein the scanning probe microscope includes a plurality of probes for detecting information about different regions of the sample surface and a set of deflection sensors for detecting deflection of each of the plurality of probes, and the method is performed with respect to a plurality of locations on the sample surface,
and preferably wherein in the scanning probe microscope, the plurality of probes for detecting information about the sample surface are arranged relative to a probe support at predetermined intervals.

11. A scanning probe microscope, comprising
a probe for detecting information about a surface of a sample, the probe including a cantilever and a cell attached thereto;
a scanner that moves the probe relative to the sample to allow the probe to scan the surface of the sample; and
a deflection sensor that detects a deflection of the probe.

12. The scanning probe microscope of claim 11, wherein the deflection sensor includes a light source that is positioned to irradiate light onto the other end of the cantilever and a position-sensitive photodetector that is positioned to detect the light reflected from the other end of the cantilever, and the scanner includes an expanding piezo-electric element that is connected to the probe,
and preferably wherein the scanning probe microscope is an atomic force microscope (AFM).

13. The scanning probe microscope of claim 16, wherein the cell is immobilized by electrostatic binding on the other end of the cantilever coated with a cationic polyelectrolyte.

14. The scanning probe microscope of claim 16, wherein the scanning probe microscope includes a plurality of probes for detecting information about different regions of the surface of the sample and a set of deflection sensors for detecting each deflection of the plurality of probes,
and preferably wherein the plurality of probes are arranged relative to a probe support at predetermined intervals.

15. A probe for detecting information about a surface of a sample, comprising:
a cantilever including a cell attached to one end thereof,
wherein the cell is attached to a cationic polyelectrolyte coated on the end of the cantilever,
and preferably wherein the cationic polyelectrolyte is poly(diallyldimethylammonium chloride) (polyDADMAC),
and further preferably wherein the cell is E.coli.
